# EUROPÄISCHE PATENTSCHRIFT

(11) **EP 0 892 647 B1**
(45) Veröffentlichungstag und Bekanntmachung des Hinweises auf die Patenterteilung: **17.09.2003**
(21) Anmeldenummer: 97920631.5
(22) Anmeldetag: 10.04.1997
(51) Int. Cl.: A61L 15/24, A43B 17/10

(54) **VERWENDUNG VON MIKROPORÖSEM POLYOLEFIN ZUR ABSORPTION VON SCHWEISS UND ANDEREN KÖRPERAUSDÜNSTUNGEN**
USE OF MICROPOROUS POLYOLEFINE FOR ABSORBING SWEAT AND OTHER BODILY EXHALATIONS
UTILISATION DE POLYOLEFINE MICROPOREUSE POUR L'ABSORPTION DE LA SUEUR ET AUTRES EXHALAISONS CORPORELLES

(30) Priorität: 12.04.1996 DE 19616224
(43) Veröffentlichungstag der Anmeldung: 27.01.1999
(73) Patentinhaber: DARAMIC, INC., North Charleston, SC 29406 (US)
(72) Erfinder: GAILLARD, Claude, F-68320 Jebsheim (FR); MEYER, Jacques, F-67600 Sélestat (FR)
(74) Vertreter: UEXKÜLL & STOLBERG
(86) Internationale Anmeldenummer: EP9701757
(87) Internationale Veröffentlichungsnummer: WO97038736

(56) Entgegenhaltungen:
- EP-A- 0 272 690
- DE-A- 1 496 123
- DE-A- 3 330 060
- DE-A- 4 213 828
- DATABASE WPI Section Ch, Week 8741 Derwent Publications Ltd., London, GB; Class A32, AN 87-288418 XP002040364 & JP 62 201 642 A (ISONO K) , 5.September 1987

## Beschreibung

Die Erfindung betrifft die Verwendung eines Materials auf Basis von mikroporösem, gefüllten Polyolefin zur Absorption von Schweiß und anderen Körperausdünstungen sowie zur Vermeidung von Geruchsbildung.

Lebewesen, insbesondere Menschen, sondern Schweiß und andere Körperausdünstungen ab, die zu einer unangenehmen Geruchsbildung führen. Ein typisches Beispiel hierfür sind die bekannten Schweißfüße, die zu einer unangenehmen Geruchsbildung im Schuhwerk führen. Ähnliche Probleme bestehen in anderen Körperbereichen und der diese Körperbereiche abdeckenden Bekleidung.

Dementsprechend hat es in der Vergangenheit nicht an Vorschlägen für Materialien gemangelt, die zur Vermeidung oder Verminderung von Körpergerüchen führen sollen. So ist beispielsweise aus der WO 88/0376 eine Schweißeinlage für Wäsche- oder Kleidungsstücke bekannt, die eine hautfreundliche Saugschicht aus Papiervlies, Faservlies, Filz, Zellstoff, Cellulosevlies oder einem Gewebe aus Wolle oder Baumwolle und eine dünne wasserundurchlässige Außenschicht aufweist, wobei die Saugschicht mikroverkapselte Duftstoffe und/oder mikroverkapselte schweißzersetzende und/oder die Schweißbildung hemmende Wirkstoffe enthält. Ferner ist aus der DD-PS 230 419 eine Einlegesohle aus einem Verbund eines extrudierten Polyolefinschaumes mit einem Textil und mit einer antimykotischen und/oder geruchsverbessernden Ausrüstung bekannt, die ein- oder beidseitig eine kompakte flexible Sperrschicht mit bestimmten physikalischen Parametern aus Duroplast, Thermoplast, Lefa oder aus verdichtetem Polyolefinschaum aufweist, die mit dem Polyolefinschaum thermisch verbunden oder verklebt ist. Weiterhin sind aus der DE-A-33 06 843 absorbierende Erzeugnisse mit einer integrierten Anordnung einer flüssigkeitsundurchdringlichen Rückschicht, einem hierauf befindlichen absorbierenden Medium sowie einer flüssigkeitsdurchdringbaren Schicht, die auf dem absorbierenden Medium aufliegt, bekannt. Die Rückschicht besteht aus einem porösen, dampfdurchdringbaren, flüssigkeitsundurchdringlichen Film, der durch Vermischen von 100 Gewichtsteilen eines Polyolefinharzes, 28 bis 200 Gewichtsteilen Füllpartikel und 10 bis 70 Gewichtsteilen eines flüssigen oder wachsähnlichen Kohlenwasserstoffpolymeren oder eines flüssigen Gummis und Ausformen der Mischung zu einem Film hergestellt ist. Dieser wird dann anschließend zur Bildung feiner Poren in dem Film um das 1,2-fache der ursprünglichen Dimension in mindestens einer Oberflächenrichtung gestreckt. Bei diesem absorbierenden Erzeugnis handelt es sich vorzugsweise um eine Wegwerfwindel oder eine Damenbinde.

Die DE-A-3 330 060 beschreibt Einlegesohlen aus einem Kunststoffformstück, das mit einer Paarung von zwei unterschiedlichen Metallen versehen ist.

Die japanischen Kokai-Veröffentlichung JP-A-62-201642 offenbart ein Material, das ein thermoplastisches Harz und ein Adsorbens umfasst, sowie die Verwendung des Materials zu Deodorisierungszwecken, beispielsweise in Kühlschränken.

Demgegenüber wurde nunmehr überraschend gefunden, daß ein Material, das schon seit Jahrzehnten als Batteriescheider in Akkumulatoren und Batterien verwendet wird, sich ausgezeichnet zur Bekämpfung von unangenehmen Gerüchen eignet, die durch Schweiß und anderen Körperausdünstungen entstehen.

Gegenstand der Erfindung ist dementsprechend die Verwendung eines Materials auf Basis von mikroporösem, gefüllten Polyolefin, das auf einem homogenen Gemisch von 15 bis 60 Vol.-% Polyolefin mit einem Molekulargewicht (Gewichtsmittel) von mindestens 300 000, einem gemäß ASTM-D-1278-57T (Bedingung E) gemessenen Standardbelastungs-Schmelzindex von kleiner als 0,1 und einer mit einer Lösung von 0,02 g des Polyolefins in 100 g Decalin bei 130°C gemessenen reduzierten Viskosität von nicht weniger als 4,0, 35 bis 80 Vol.-% feinteiligem Siliciumdioxid als Füllstoff und 1 bis 10 Vol.-% Weichmacher basiert und eine Porengröße von kleiner als 1 µm Porendurchmesser aufweist, zur Absorption von Schweiß und anderen Körperausdünstungen.

Gegenstand der Erfindung sind ferner Schuhe, Bekleidungsstücke, Unterlagen, Auskleidungen, Einlagen und deren Teile, die das oben genannte Material auf Basis von mikroporösem, gefüllten Polyolefin enthalten oder daraus bestehen.

Bevorzugte Ausführungsformen und Vorteile der Erfindung ergeben sich aus der nachfolgenden Beschreibung sowie den Unteransprüchen.

Wenngleich das erfindungsgemäß verwendete Material offensichtlich für eine Vielzahl von Anwendungen geeignet ist und in seiner Ausbildung an diese Anwendungen angepaßt werden kann, liegt das Hauptanwendungsgebiet zur Zeit im Schuhbereich, insbesondere als Sohle oder Einlegesohle. Deshalb wird die Erfindung im folgenden hauptsächlich anhand dieser bevorzugten Ausführungsform erläutert, ohne daß dies als Einschränkung aufzufassen ist.

Wie bereits eingangs erwähnt, ist das erfindungsgemäß verwendete Material auf Basis von mikroporösem, gefüllten Polyolefin bereits seit langem als Batteriescheidermateriali bekannt und ausführlich in der US-PS 3 351 495 und der entsprechenden DE-AS 1 496 123 beschrieben. Bei dem Polyolefin handelt es sich um ultrahochmolekulares Polyolefin, vorzugsweise ultrahochmolekulares Polyethylen. Es besitzt ein mittleres gewichtsmäßiges Molekulargewicht von mindestens 300 000, vorzugsweise mindestens 1 000 000 und insbesondere etwa 4 bis 7 × 10⁶. Der Standardbelastungs-Schmelzindex des Polyolefins beträgt im wesentlichen 0, d.h. er ist kleiner als 0,1 und vorzugsweise kleiner als 0,01. Die reduzierte Viskosität des Polyolefins beträgt nicht weniger als 4,0 und liegt vorzugsweise über 10 und insbesondere über 15. Hinsichtlich der Bestimmung von Standardbelastungs-Schmelzindex und reduzierter Viskosität sei auf die oben genannte US-PS 3 351 495 bzw. die DE-AS 1 496 123 verwiesen. Wie in diesen Druckschriften erläutert können auch Polyolefingemische verwendet werden. Neben Polyethylen sind insbesondere auch Polypropylen, Polybuten, Polystyrol, Ethylen-Propylen-Copolymere, Ethylen-Hexylen-Copolymere, Ethylen-Buten-Copolymere, Propylen-Buten-Copolymere, Ethylen-Propylen-Buten-Copolymere und Copolymere aus Ethylen oder Propylen mit einer ethylenisch ungesättigten Monocarbonsäure, nämlich Acrylsäure, Methacrylsäure oder deren Gemische geeignet.

Geeignete Füllstoffe und Weichmacher sind dem Fachmann bekannt. Hierzu wird wiederum auf die US-PS 3 351 495 und die DE-AS 1 496 123 verwiesen. Ein bevorzugter Füllstoff ist feinteiliges Siliciumdioxid (Kieselsäure). Die durchschnittliche Teilchengröße (Durchmesser) des Füllstoffs liegt im Bereich von 0,01 bis etwa 20 µm, wobei die Oberfläche des Füllstoffs im Bereich von 30 bis 950 m²/g liegt und vorzugsweise mindestens 100 m²/g beträgt.

Als Weichmacher enthält das erfindungsgemäß zu verwendende Material vorzugsweise ein wasserunlösliches Öl, insbesondere Prozeßöl.

Erfindungsgemäße Mengenbereiche für das homogene Gemisch sind 15 bis 60 Vol.-% Polyolefin, insbesondere 30 bis 45 Vol.-% Polyolefin, 35 bis 80 Vol.-% Füllstoff, insbesondere 50 bis 65 Vol.-% Füllstoff, und 1 bis 10 Vol.-% Weichmacher.

Außer den genannten Bestandteilen kann das erfindungsgemäß zu verwendende Material übliche Zusätze wie Antioxidantien (gewöhnlich 0,1 bis 1 %), Gleitmittel (gewöhnlich 0,1 bis 1 %), Antistatika, Pigmente, Farbstoffe, leitfähigen Ruß, Stabilisatoren, Lichtschutzmittel und dergleichen enthalten.

In einer besonders bevorzugten Ausführungsform wird das erfindungsgemäß zu verwendende Material mit antibakteriellen und/oder fungiziden Mitteln behandelt. Diese können in beliebiger Form aufgebracht werden. Bevorzugt ist es, diese Mittel in Form einer wäßrigen Lösung aufzusprühen oder aufzucoaten. Die jeweilig aufzubringende Menge richtet sich nach der Anwendung des erfindungsgemäß zu verwendenden Materials und liegt üblicherweise bei 1 bis 10 g/m² Oberfläche, vorzugsweise etwa 5 g/m² Oberfläche. Dabei hat sich die Mitverwendung von Tensiden als vorteilhaft erwiesen. Diese fördern die Benetzung und damit die gleichmäßige Verteilung auf der Oberfläche und tragen erheblich zur Saugfähigkeit des Materials und damit zur wirksamen Geruchsbekämpfung bei. Besonders geeignet sind Tenside mit der Formel R-O-R₁, in der R ein verzweigter oder geradkettiger, substituierter oder nicht-substituierter Alkyl-, Alkenyl- oder Alkinylrest mit 6 bis 50 Kohlenstoffatomen und R₁ ein Oxyalkyl-, Glycerinoxyalkyl- oder Sorbitanoxalkylrest ist, wobei der Oxalkylierungsgrad 2 bis 80 beträgt. Ein erfindungsgemäß gut geeignetes Beispiel ist das Tensid mit der Formel R-O-(C₂H₄O)ₓ-(C₃H₆O)_{y}-H, wobei R ein C₁₂H₂₅ bis C₁₈H₃₇-Rest ist, x 8 beträgt und y 6 bis 10 beträgt. Derartige Tenside sind im Handel unter der Bezeichnung PLURAFAC® LF 700 von der Fa. BASF erhältlich.

Insbesondere bei Verwendung im Schuhbereich hat sich eine Zusammensetzung besonders bewährt, die aus 25 bis 35 Gew.-% einer Lösung des oben genannten Tensids in wäßrigem Isopropylalkohol (28 Gew.-% Tensid, 40 Gew.-% Isopropanol und 32 Gew.-% Wasser), 1,5 bis 2,5 Gew.-% Pfefferminzöl, 0,5 bis 1,5 Gew.-% Zinksulfat, 3 bis 5 Gew.-% Natriumhypochlorit, 2 bis 5 Gew.-% Campher und im übrigen Wasser besteht.

Das erfindungsgemäße Material wird vorzugsweise in der in der US-PS 3 351 495 bzw. der DE-AS 1 496 123 beschriebenen Weise, insbesondere durch Extrusion und anschließende Extraktion hergestellt (siehe auch EP 0 425 784 B1). Dabei ist es bevorzugt, daß das erfindungsgemäße Material in Form einer Bahn extrudiert wird, aus der dann die gewünschten Formen ausgeschnitten werden. Das Besprühen oder Beschichten mit der Lösung der antibakteriellen und fungiziden Mittel kann im Anschluß an die Extraktion der Bahn oder aber auch nach dem Herausschneiden der gewünschten Formen aus der Bahn erfolgen. Bezüglich der Einzelheiten der Extrusion, der Extraktion und der damit beeinflußbaren Porosität sei auf die vorgenannten Druckschriften verwiesen.

Das erfindungsgemäß zu verwendende Material weist eine Porengröße von kleiner als 1 µm Porendurchmesser und insbesondere kleiner als 0,5 µm Porendurchmesser auf. Vorzugsweise besitzen mehr als 50 % der Poren einen Durchmesser von 0,5 µm oder weniger. Als gut geeignet haben sich Materialien mit einer mittleren Porengröße im Bereich von etwa 0,10 bis 0,15 µm erwiesen. Das Leervolumen (Porosität) beträgt vorzugsweise mindestens 50 % und insbesondere mindestens 55 %, beispielsweise 57 bis 65 %. Es kann aber auch bis zu 70 oder sogar 80 % betragen.

Bei Verwendung als Schuhmaterial, insbesondere als Einlegesohle, weist das bahnförmige Material vorzugsweise zumindest auf einer Seite Rippen auf, die aus demselben oder einem anderen Material wie das erfindungsgemäß zu verwendende Material bestehen können. Bevorzugt sind parallel zueinander verlaufende Rippen in einem Abstand von etwa 3 bis 4 mm. Ähnlich gut geeignet sind aber auch zick-zackförmig oder sinusförmig verlaufende Rippen. Bei Verwendung als Einlegesohle sollen diese Rippen quer zur Längsachse der Einlegesohle verlaufen. Bei zick-zackförmigen oder sinusförmigen Rippen sind in Querrichtung zur Längsache der Einlegesohle im allgemeinen 0,5 bis 5 Perioden der Zick-Zack- oder Wellenform vorhanden. Die Rippen verbessern nicht nur die mechanischen Eigenschaften des bahnförmigen Materials, sondern führen auch zu einer guten Durchlüftung, beispielsweise im Falle einer Einlegesohle zwischen der Einlegesohle und dem Untergrund. Durch das Zusammenwirken von Porosität des erfindungsgemäßen verwendeten Materials und der durch die Rippen ausgebildeten, belüfteten Hohlräume wird ein Schwammeffekt vermieden, so daß die Körperausdünstungen wahrscheinlich im wesentlichen zwischen den Rippen eintrocknen. Außerdem bildet sich dadurch eine Luftschicht die zu einer Wärmeisolierung führt, so daß die Füße bei Kälte warm und bei großer Wärme kühl bleiben.

Im Falle, daß das erfindungsgemäß zu verwendende Material in Form einer Bahn vorliegt, beträgt die Dicke etwa 0,025 bis 1,5 mm, vorzugsweise etwa 0,2 bis 1 mm und insbesondere etwa 0,3 bis 0,8 mm (z.B. etwa 0,5 mm). Die Höhe der Rippen hängt von der Dicke der Materialbahn, der gewünschten Anwendung und der gewünschten mechanischen Stabilität ab. Bei Verwendung als Einlegesohle liegt die Gesamtdicke des Materials (Bahn plus Rippen) üblicherweise bei etwa 2 mm.

Es ist offensichtlich, daß das erfindungsgemäß zu verwendende Material überall dort angewendet werden kann, wo Schweiß und andere Körperausdünstungen zu erwarten sind. Bevorzugte Anwendungsgebiete sind dementsprechend Schuhe, Bekleidungsstücke, Unterlagen (für Mensch und Tier), Auskleidungen (beispielsweise von Hundehütten und ähnlichen Behältnissen für den Aufenthalt oder Transport von Tieren), Einlagen und deren Teile. Dabei kann die Form des erfindungsgemäß zu verwendende Material an den jeweiligen Verwendungszweck angepaßt werden. Bei Verwendung in Bekleidungsstücken bietet sich beispielsweise die Form kleiner Polster an, die in die Bekleidungsstücke herausnehmbar bzw. austauschbar eingearbeitet werden können. Für die meisten Verwendungszwecke ist allerdings das oben beschriebene bahn- oder bogenförmige Material, gegebenenfalls mit ein- oder beidseitigen Rippen, bevorzugt.

### Beispiel

Es wurde ein bahnförmiges Material durch Extrusion und anschließende Extraktion wie in der US-PS 3 351 495 und der DE-AS 1 496 123 beschrieben hergestellt. Das erhaltene Produkt bestand aus 51 Vol.% Siliciumdioxid, 40 Vol.-% ultrahochmolekularem Polyethylen mit einem durchschnittlichen Molekulargewicht von 5,6 x 10⁶, 2,5 Vol.-% Ruß (Füllstoff) und 0,5 Vol.-% Antioxidans. Der Restgehalt an Weichmacher (Prozeßöl) betrug 6 Vol.-%. Durch Verwendung geeigneter Walzen wurden auf der einen Seite des bahnförmigen Materials parallele, in einem Abstand von etwa 3 mm verlaufende Rippen erzeugt. Das bahnförmige Material besaß eine Dicke von etwa 0,5 mm, während die Rippen eine Höhe von etwa 1,5 mm aufwiesen.

Die mittlere Porengröße des so hergestellten Produkts wurde durch Quecksilber-Porosimetrie zu 0,125 µm bestimmt. Das Leervolumen (Porosität) betrug etwa 64 %.

Aus diesem bahnförmigen Material wurden Einlegesohlen herausgeschnitten und mit einer Lösung besprüht, die die oben angegebene Zusammensetzung aus Tensid, Pfefferminzöl, Zinksulfat, Natriumhypochlorit, Campher und Wasser besaß. Es wurden etwa 5 g/m² dieser Lösung aufgesprüht.

Die so hergestellten Einlegesohlen wurden über einen längeren Zeitraum von 30 Personen verwendet. Alle Personen berichteten übereinstimmend, daß sie keine oder zumindest eine sehr stark verringerte Geruchsbildung beim Tragen dieser Einlegesohlen beobachteten.

## Patentansprüche

1. Verwendung eines Materials auf Basis von mikroporösem, gefüllten Polyolefin, das auf einem homogenen Gemisch von 15 bis 60 Vol. -% Polyolefin mit einem Molekulargewicht (Gewichtsmittel) von mindestens 300 000, einem gemäß ASTM-D-1278-57T (Bedingung E) gemessenen Standardbelastungs-Schmelzindex von kleiner als 0,1 und einer mit einer Lösung von 0,02 g des Polyolefins in 100 g Decalin bei 130°C gemessenen reduzierten Viskosität von nicht weniger als 4,0, 35 bis 80 Vol.-% feinteiligem Siliciumdioxid als Füllstoff und 1 bis 10 Vol.-% Weichmacher basiert und eine Porengröße von kleiner als 1 µm Porendurchmesser aufweist, zur Absorption von Schweiß und anderen Körperausdünstungen.

2. Verwendung nach Anspruch 1, bei der das Polyolefin Polyethylen, vorzugsweise ultrahochmolekulares Polyethylen ist.

3. Verwendung nach Anspruch 1 oder 2, bei der mehr als 50% der Poren des Materials einen Durchmesser von 0,5 µm oder weniger aufweisen.

4. Verwendung nach einem der Ansprüche 1 bis 3, bei der das Leervolumen des Materials mindestens 50% beträgt.

5. Verwendung nach einem der Ansprüche 1 bis 4, bei der der Weichmacher Prozessöl ist.

6. Verwendung nach einem der Ansprüche 1 bis 5, bei der das Material als bahn- oder bogenförmiges Material oder daraus ausgeschnittenen Formteilen verwendet wird.

7. Verwendung nach Anspruch 6, bei der das bahn- oder bogenförmige Material zumindest auf einer Seite parallel zueinander verlaufende Rippen aufweist.

8. Verwendung nach einem der Ansprüche 1 bis 7, bei der das Material mit antibakteriellen und/oder fungiziden Mitteln behandelt worden ist.

9. Verwendung nach Anspruch 8, bei der das Material mit einer Zusammensetzung behandelt worden ist, die aus einem Tensid mit der Formel R-O-R₁, in der R ein verzweigter oder geradkettiger, substituierter oder nicht-substituierter Alkyl-, Alkenyl- oder Alkinylrest mit 6 bis 50 Kohlenstoffatomen und R₁, ein Oxylalkyl-, Glycerinoxyalkyl- oder Sorbitanoxalkylrest ist, Pfefferminzöl, Zinksulfat, Natriumhypochlorit, Campher, Wasser und gegebenenfalls einer geringen Menge organischem Lösungsmittel besteht.

10. Verwendung nach einem der Ansprüche 1 bis 9, bei der das Material zur Absorption von Schweiß und anderen Körperausdünstungen in Schuhen, Bekleidungsstücken, Unterlagen, Auskleidungen, Einlagen und deren Teile verwendet wird.

11. Schuhe, Bekleidungsstücke, Unterlagen, Auskleidungen, Einlagen und deren Teile, die das in den Patentansprüchen 1 bis 9 definierte Material enthalten oder daraus bestehen.

12. Teil nach Anspruch 11, das eine Sohle oder eine Einlegesohle eines Schuhs ist.

## Claims

1. Use of a material based on microporous, filler-containing polyolefin, which is based on a homogeneous mixture of 15 to 60 vol.% polyolefin having a molecular weight (weight-average) of at least 300,000, a standard load melt index, measured in accordance with ASTM-D-1278-57T (condition E), of less than 0.1 and a reduced viscosity, measured with a solution of 0.02 g of the polyolefin in 100 g decalin at 130°C, of not less than 4.0, 35 to 80 vol.% finely divided silicon dioxide as filler and 1 to 10 vol.% plasticizer and has a pore size of less than 1 µm pore diameter, for absorbing sweat and other bodily exhalations.

2. Use according to claim 1, in which the polyolefin is polyethylene, preferably ultra-high molecular weight polyethylene.

3. Use according to claim 1 or 2, in which more than 50% of the pores of the material have a diameter of 0.5 µm or less.

4. Use according to one of claims 1 to 3, in which the void volume of the material is at least 50%.

5. Use according to one of claims 1 to 4, in which the plasticizer is process oil.

6. Use according to one of claims 1 to 5, in which the material is used as a web or sheet material or shapes cut out therefrom.

7. Use according to claim 6, in which the web or sheet material has, at least on one side, ribs running parallel to one another.

8. Use according to one of claims 1 to 7, in which the material has been treated with antibacterial and/or fungicidal agents.

9. Use according to claim 8, in which the material has been treated with a composition which consists of surfactant of the formula R-O-R₁, in which R is a branched or straight-chain, substituted or unsubstituted alkyl, alkenyl or alkinyl radical having 6 to 50 carbon atoms and R₁ is an oxyalkyl, glyceroloxyalkyl or sorbitanoxyalkyl radical, peppermint oil, zinc sulphate, sodium hypochlorite, camphor, water and, if appropriate, a small amount of organic solvent.

10. Use according to one of claims 1 to 9, in which the material for absorbing sweat and other bodily exhalations is used in shoes, articles of clothing, underlays, linings, inlays and parts thereof.

11. Shoes, articles of clothing, underlays, linings, inlays and parts thereof which comprise the material defined in patent claims 1 to 9 or consist of this material.

12. Part according to claim 1, which is a sole or an inner sole of a shoe.

## Revendications

1. Utilisation d'un matériau à base de polyoléfine microporeuse contenant une matière de charge, qui repose sur un mélange homogène comprenant 15 à 60 % en volume de polyoléfine avec un poids moléculaire (moyenne de poids) d'au moins 300 000, un indice de fusion sous charge standard mesuré selon ASTM-D-1278-57T (condition E) inférieur à 0,1 et une viscosité réduite non inférieure à 4,0, mesurée à 130° C avec une solution de 0,02 g de polyoléfine dans 100 g de décaline, 35 à 80 % en volume de dioxyde de silicium finement divisé formant la matière de charge et 1 à 10 % en volume de plastifiant et qui possède une taille de pore avec un diamètre de pore inférieur à 1 µm, destiné à absorber la transpiration et autres émanations du corps.

2. Utilisation selon la revendication 1, dans laquelle la polyoléfine est un polyéthylène, de préférence un polyéthylène de masse moléculaire ultra élevée.

3. Utilisation selon la revendication 1 ou 2, dans laquelle plus de 50 % des pores du matériau ont un diamètre de 0,5 µm ou moins.

4. Utilisation selon l'une quelconque des revendications 1 à 3, dans laquelle le volume vide du matériau est de l'ordre de 50 % au moins.

5. Utilisation selon l'une quelconque des revendications 1 à 4, dans laquelle le plastifiant est l'huile de traitement.

6. Utilisation selon l'une quelconque des revendications 1 à 5, dans laquelle le matériau est utilisé sous forme de matériau en bande ou en feuille ou sous forme de pièces formées découpées dans celui-ci.

7. Utilisation selon la revendication 6, dans laquelle le matériau en bande ou en feuille comporte au moins sur une face des nervures orientées parallèlement les unes aux autres.

8. Utilisation selon l'une quelconque des revendications 1 à 7, dans laquelle le matériau a été traité avec des agents antibactériens et/ou fongicides.

9. Utilisation selon la revendication 8, dans laquelle le matériau a été traité avec une composition, qui est formée par un agent tensioactif selon la formule R-O-R₁, dans laquelle R est un alkyle, un alkényle ou un alkinyle droit ou ramifié, substitué ou non substitué, avec 6 à 50 atomes de carbone, et R₁ est un oxyalkyle, un oxyalkyle glycérique ou un oxyalkyle de sorbitan, par l'essence de menthe poivrée, le sulfate de zinc, l'hypochlorite de sodium, le camphre, l'eau et, le cas échéant, une faible quantité de solvant organique.

10. Utilisation selon l'une quelconque des revendications 1 à 9, dans laquelle le matériau est utilisé pour absorber la transpiration et autres émanations du corps dans des chaussures, des vêtements, des supports, des revêtements, des semelles intérieures ou des éléments de celles-ci.

11. Chaussures, vêtements, supports, revêtements, semelles intérieures ou éléments de celles-ci, qui contiennent le matériau défini selon les revendications 1 à 9 ou sont réalisés dans celui-ci.

12. Élément selon la revendication 11 qui est une semelle ou une semelle intérieure d'une chaussure.
